# EUROPEAN PATENT SPECIFICATION

(11) **EP 2 394 677 B1**
(45) Date of publication and mention of the grant of the patent: **02.01.2013**
(21) Application number: 10165307.9
(22) Date of filing: 08.06.2010
(51) Int. Cl.: A61F 13/15, A61M 1/00, A61M 27/00

(54) **Medical product, in particular for removing body liquids from human and/or animal body cavities**
Medizinisches Produkt, insbesondere zum Entfernen von Körperflüssigkeiten aus menschlichen und/oder tierischen Körperhöhlen
Produit médical, en particulier pour enlever des liquides corporels de cavités corporelles humaines et/ou animales

(43) Date of publication of application: 14.12.2011
(73) Proprietor: Aesculap AG, 78532 Tuttlingen/Donau (DE)
(72) Inventor: Siedle, Gabriel Dr., 79117 Freiburg (DE); Abele, Wolfgang Dr., 78532 Tuttlingen (DE); Odermatt, Erich Dr., 8200 Schaffhausen (CH)
(74) Representative: Patentanwälte Ruff, Wilhelm, Beier, Dauster & Partner

(56) References cited:
- EP-A1- 2 098 257
- DE-A1-102008 061 535
- DE-A1-102008 061 536
- GB-A- 201 834

## Description

The present invention is related to a medical product which is suitable in particular for removing body liquids from human and/or animal body cavities.

One possible way of treating infected or infectious body cavities is provided by the so-called endoluminal vacuum therapy. This form of therapy employs a particular wound drainage system which is generally composed of an essentially open-pore sponge for absorbing wound fluids and secretions and a drainage tube for removing or discharging the absorbed wound fluids. Such a wound drainage system is marketed by B. Braun Aesculap AG under the name "Endosponge", for example.

In general, the sponge of the wound drainage system is placed into the body cavity to be treated by means of a tubular or hose type insertion instrument and a slider instrument (pusher) to push the sponge through the insertion instrument. Typically, the sponge is adapted to reversible compression, and usually has a volume expansion larger than the internal diameter of the insertion instrument. Thus, the sponge has to be pushed through the insertion instrument in a compressed form. To facilitate this cumbersome procedure for an operator, the sponge may be provided with a lubricant prior to transfer into the insertion instrument. However, such a modified procedure will soon meet its limits, in particular with small diameter insertion instruments.

Another drawback is that the sponge will expand immediately after its ejection from the insertion instrument, where a necessary repositioning or replacing of the ejected sponge would be feasible but with difficulties or not at all.

EP 2 098 257 A1 discloses a wound dressing for use in a vacuum wound therapy treatment. The wound dressing comprises a backing layer to serve as a fluid barrier, a contact layer and a cover layer.

The post-published German patent applications DE 10 2008 061 536.6 and DE 10 2008 061 535.8 disclose wound drainage systems for endoluminal vacuum therapy, each including a repositionable absorbent medical body. The absorbent body described in DE 10 2008 061 536.6 is maintained in a compressed form by means of a wrapping. However, a basic insufficiency related therewith is that the wrapping has to be removed by an operator to allow expansion of the absorbent body. With the absorbent body described in DE 10 2008 061 535.8 compression is effected by means of an impregnation comprising a material that will be removed upon contact with a biocompatible liquid. The relatively cumbersome production of the compressed absorbent body is an adverse fact. To effect compression, the absorbent body is in general transferred into a tube made of aluminium or stainless steel, and subsequently impregnated with a solution or suspension of the impregnation material to be applied to the absorbent body.

GB 201,834 discloses an absorbent body, wherein a film made of starch, vegetable gum, gelatine or the like may be provided.

Thus, the present invention is aimed at the problem to provide a medical product based on an absorbent body for body liquids to prevent the above mentioned disadvantages of the state of the art, and in particular to allow a controlled deployment, unfolding or expansion of the absorbent body, in particular after placement of the absorbent body in a human and/or animal body cavity.

The problem is solved according to the invention by a medical product, in particular for removing body liquids from human and/or animal body cavities, comprising an absorbent body, a drainage tube, and a wrapping surrounding the absorbent body and keeping the absorbent body in a compressed form. The wrapping comprises at least one water-soluble layer, in particular an internal layer, preferably immediately surrounding the absorbent body, and an external layer, wherein at least one of the layers, i.e. the internal layer and/or external layer, is water-soluble.

The medical product according to the present invention may advantageously be employed for discharging or removing pathological body liquids from human and/or animal body cavities. The medical product is preferably used in the scope of so-called endoluminal vacuum therapy which will be described in more detail below. The medical product may, for example, be used for treating intra-abdominal or intracavity abscesses, fistulas, pancreatitis or the like. Regarding the fistulas, the medical product may be employed for the treatment of fistulas of small intestine and/or gall bladder.

One of the main advantages of the medical product is that the absorbent body may be flushed with water or an aqueous medium directly from inside via the drainage tube that preferably invades the absorbent body to effect dissolution of the at least one water-soluble layer of the wrapping. With progressing dissolution of the at least one water-soluble layer, there is an increasing degradation and weakening of the wrapping, whereby the ability of the wrapping to counteract the unfolding or expansion tendency of the absorbent body is decreasing. Finally, when the pressure of the absorbent body acting on the wrapping is higher than the counter pressure of the wrapping, the wrapping will yield, in particular the remaining wrapping rips open, whereby the absorbent body may completely unfold or expand. This allows for a time-controllable decomposition or degradation, in particular dissolution, of the wrapping and thus for a time-controllable unfolding or expansion of the absorbent body. Due to the timely delayed decomposition of the wrapping, and thus timely delayed unfolding of the absorbent body, it is possible for an operator to replace the medical product within a body cavity to be treated, if a replacement is recommendable in view of therapeutic terms.

Within the scope of the present invention, the expression "pathological body liquids" are preferably understood as wound secretions, exudates, abscess fluids or the like.

Within the scope of the present invention, the expression "body cavities" are preferably so-called wound cavities. This is preferably intended to mean naturally occurring or pathologically induced protuberances, generally of hollow organs, like blood vessels, large intestine, small intestine, gall bladder, esophagus, urethra etc.. For example, said protuberances may be present as diverticals, aneurysms, fistulas and/or abscesses within the body of a patient. The protuberances may become filled with wound secretions. The pathologically induced protuberances are often the result of operational interventions, in particular anastomoses. Protuberances may be formed there in the ligature or suture region, and may rapidly become larger owing to infection. Protuberances in the region of the suturing are often also referred to as so-called insufficiency cavities.

Within the scope of the present invention, the expression "absorbent body" preferably means a body that is capable of absorbing body liquids, in particular pathological body liquids such as wound secretions.

Within the scope of the present invention, the expression "wrapping" may be understood as a synonym for envelope or encasing.

Within the scope of the present invention, the expression "drainage tube" preferably refers to a tube that is capable of removing body liquids absorbed by the absorbent body from the absorbent body and that is capable of delivering a liquid such as water or an aqueous salt, buffer or electrolyte solution to the inside of the absorbent body, preferably in order to flush the absorbent body from the inside thereof.

Within the scope of the present invention, the expression "water-soluble layer" or "water- soluble component" is preferably to comprehend a layer or component that is at least partially soluble, preferred completely soluble, in water or an aqueous medium, in particular an aqueous solution, like an aqueous salt, buffer or electrolyte solution, for example and/or body liquids, like blood, wound secretions, lymph or the like, for example. The dissolution process of the water-soluble layer or component may be accompanied by a swelling process, as the case may be.

Within the scope of the present invention, the expression "at least one water-soluble layer" preferably encompasses one water-soluble layer or a plurality of water-soluble layers, in particular two water-soluble layers.

In principle, the wrapping may only partly surround the absorbent body. However, it is more preferably, that the wrapping completely surrounds the absorbent body, preferably apart from an area of the absorbent body where the drainage tube preferably emerges out of or protrudes from the absorbent body.

In a preferred embodiment, the internal layer, more preferably only the internal layer, of the wrapping is water-soluble. The advantage thereof is that the absorbent body may be flushed with water or an aqueous medium directly from inside via the drainage tube that preferably invades the absorbent body to effect dissolution of the internal layer of the wrapping. With progressing dissolution of the internal layer, there is an increasing degradation and weakening of the wrapping, whereby the ability of the wrapping to counteract the unfolding or expansion tendency of the absorbent body is decreasing. Finally, when the pressure of the absorbent body acting on the wrapping is higher than the counter pressure of the wrapping, the wrapping will yield, in particular the remaining wrapping rips open, whereby the absorbent body may completely unfold or expand. This allows for a time-controllable decomposition or degradation, in particular dissolution, of the wrapping and thus for a time-controllable unfolding or expansion of the absorbent body. Due to the timely delayed decomposition of the wrapping, and thus timely delayed unfolding of the absorbent body, it is possible for an operator to replace the medical product within a body cavity to be treated, if a replacement is recommendable in view of therapeutic terms. Further, the water-solubility characteristics of the external layer preferably prevents from a premature decomposition of the wrapping from the outside thereof, and thus from a premature unfolding of the absorbent body. Thus, the operator, at least within a certain time limit, may determine at which time decomposition of the wrapping, and consequently unfolding of the absorbent body shall be initiated by flushing the absorbent body from inside via the drainage tube. A further advantage is that it is not necessary any longer for the operator to remove, in particular to pull away, the wrapping from the absorbent body.

In a further embodiment, the at least one water-soluble layer, preferably the internal layer, more preferably only the internal layer, of the wrapping comprises a water-soluble component.

The expression "a water-soluble component" as used according to the present invention may not only encompass a single water-soluble component, but may also encompass multiple water-soluble components, i. e. at least two water-soluble components, in particular two, three or more water-soluble components.

Preferred is that the internal layer and/or the external layer, in particular the internal layer and the external layer, of the wrapping are water-soluble.

In particular, the at least one water-soluble layer, preferably the internal layer and/or the external layer, more preferably the internal layer and the external layer, of the wrapping may each comprise a water-soluble component.

Particularly preferred is that the internal layer and the external layer of the wrapping differ in water-solubility characteristics. In particular, the internal layer and the external layer of the wrapping may each comprise a water-soluble component differing in water-solubility characteristics. In this manner, a time-controlled decomposition, in particular dissolution, of the wrapping, and thus time-controlled deployment or expansion of the absorbent body may be obtained.

In a particularly preferred embodiment, the internal layer has a water-solubility superior to that of the external layer of the wrapping. In this way, an operator is enabled to flush the absorbent body, after correct placement and positioning of the latter in a body cavity, directly from inside via the drainage tube. In particular, aqueous flushing liquids, like aqueous salt, buffer or electrolyte solutions, for example, may be used for flushing the internal layer. By means of said internal flushing, the internal layer may dissolve more rapidly than the external layer of the wrapping. By doing so, the wrapping will be subject to an overall decomposition and weakening. With progressing dissolution of the internal layer, the rest of the wrapping, in particular the external layer thereof, will in an increasingly lesser extent be able to maintain the absorbent body in a compressed form. Due to a deployment or expansion tendency of the absorbent body, the rest of the wrapping would finally yield and preferably rip open, whereby the absorbent body is released to expand completely in the body cavity. In that context, the external layer having a lesser water-solubility as compared to the internal layer, may be beneficial to prevent an undesirable premature decomposition, in particular dissolution, of the wrapping from the outside, and thus an undesirable premature unfolding of the absorbent body. Thereby, any necessary replacement or repositioning of the absorbent body, after placement thereof in a body cavity, may readily be performed. Furthermore, the different water-solubility characteristics of the internal and external layers advantageously allow a time-controlled or -directed decomposition or degradation, in particular dissolution, of the wrapping, and thus also a time-controlled or -directed deployment, unfolding or expansion of the absorbent body. In this way, an operator may achieve a time optimized deployment of the absorbent body. Finally, there is no longer need for removal of the wrapping by the operator.

In a further embodiment, the internal layer comprises a water-soluble component having a water-solubility superior to that of a water-soluble component of the external layer.

Preferably, the internal layer of the wrapping is formed in such a way that upon contact with water or an aqueous medium, within 10 to 70 seconds, it has dissolved at least to an extent that the wrapping may no longer contain the absorbent body enclosed therein and will decompose, in particular rip open, to allow complete expansion of the absorbent body. In particular the internal layer of the wrapping may be formed in such a way that upon contact with water or an aqueous medium, within 10 to 70 seconds, it has dissolved completely, i. e. is present in a dissolved condition.

In an advanced embodiment, the wrapping, in particular the external layer thereof, may be formed in such a way that upon contact with water or an aqueous medium, in particular upon contact with body liquids, like wound secretions for example, the wrapping is capable of keeping the absorbent body in the compressed form, at least for two minutes.

In order to provide the internal and external layers, in particular water-soluble components thereof, with different water-solubility characteristics, there are different embodiments conceivable within the scope of the present invention. Preferred embodiments are detailed in the following.

According to a further embodiment, the internal and external layers of the wrapping comprise water-soluble components having different molecular weights. Preferably, the external layer comprises a water-soluble component having a higher molecular weight than a water-soluble component of the internal layer.

According to a further suitable embodiment, the internal and external layers, in particular water-soluble components of the internal and external layers, have a different degree of hydrolyzation. Within the scope of the present invention, the expression "degree of hydrolyzation" is preferably to comprehend as so called saponification. More specifically, the external layer, in particular a water-soluble component thereof, may have a higher degree of hydrolyzation than the internal layer, in particular than a water-soluble component thereof.

In an advanced embodiment, the external layer, in particular a water-soluble component thereof, is in an at least partially hydrolyzed condition, and the internal layer, in particular a water-soluble component thereof, is in a non-hydrolyzed condition.

Further, the internal and external layers of the wrapping, in particular water-soluble components thereof, may be provided in a different degree of cross-linking. Preferably the external layer, in particular a water-soluble component of the external layer, has a higher degree of cross-linking than the internal layer, in particular a water-soluble component of the internal layer.

Particularly preferred, the external layer, in particular a water-soluble component of the external layer, is cross-linked, whereas the internal layer, in particular a water-soluble component of the internal layer, is preferably non-cross-linked. The external layer, in particular a water-soluble component thereof, may preferably be chemically cross-linked. For example, the external layer, in particular a water-soluble component thereof, may be cross-linked by a chemical cross-linking agent selected from the group consisting of formaldehyde, dialdehyde, like glutaraldehyde, polyaldehydes, diisocyanates, dicarbodiimides, and combinations thereof, for example. The external layer, in particular a water-soluble component thereof, may comprise a proportion of a chemical cross-linking agent from 3 to 30 % by weight, in particular from 5 to 25 % by weight, preferred from 10 to 20 % by weight, based on the total weight of the external layer, in particular a water-soluble component thereof.

As an alternative or in combination therewith, the external layer, in particular a water-soluble component thereof, may be physically cross-linked, in particular as a result of irradiation, like gamma-irradiation and/or a sequence of freeze-thaw cycles, for example.

In another advantageous embodiment, the internal and external layers of the wrapping have a different degree of heat treatment. Different degrees of heat treatment may commonly be obtained using different heat treatment temperatures and/or heat treatment times. It is preferred that the external layer has a higher degree of heat treatment than the internal layer.

Preferably, the external layer, in particular only the external layer, is thermally dried and heat treated, respectively. A thermally drying may be performed in a temperature range from 70 to 140 °C, in particular from 90 to 130 °C, preferred form 100 to 110 °C, for example.

In a further advantageous embodiment, the internal and external layers have a different degree of crystallinity. Preferably, the external layer, in particular only the external layer, is crystallized, in particular thermally crystallized.

To effect a time controlled dissolution of the wrapping, the internal layer and the external layer of the wrapping may further be provided in different layer thicknesses.

As a principle, the at least one water-soluble layer, in particular the internal layer and/or external layer, of the wrapping may have a layer thickness from 0.01 mm to 0.5 mm, preferred from 0.03 mm to 0.1 mm. According to the invention, it is particularly preferred that the internal layer of the wrapping has a larger layer thickness than the external layer of the wrapping.

In principle, the internal layer, in particular a water-soluble component thereof, may be made of a different material than the external layer, in particular a water-soluble component of the external layer. However, it may be preferred according to the invention that the internal layer, in particular a water-soluble component thereof, is made of a same material as the external layer, in particular a water-soluble component thereof. More preferably, the material for the internal layer, in particular a water-soluble component thereof, and for the external layer, in particular a water-soluble component thereof, may differ in view of its molecular weight, degree of hydrolyzation, degree of cross-linking and/or degree of crystallinity. The material is preferably a polymer, in particular a biopolymer.

As a particular advantage, the water-soluble component as mentioned in the preceding embodiments may be made of a material suitable for adhesion prophylaxis.

In a preferred embodiment, the at least one water-soluble layer, in particular the internal and/or external layers, of the wrapping comprises/comprise, in particular is/are made of, a water-soluble component made of a material selected from the group consisting of polyvinyl alcohol (PVA), polysaccharides such as mucopolysaccharides, alkyl celluloses, hydroxyalkyl celluloses, carboxyalkyl celluloses, salts thereof, derivatives thereof and mixtures thereof.

More preferably, the at least one water-soluble layer, in particular the internal and/or external layers, of the wrapping comprises/comprise, in particular is/are made of, a water-soluble component made of a material selected from the group consisting of polyvinyl alcohol, polyethylene glycol, gelatine, starch, amylose, amylopectin, dextran, methyl cellulose, hydroxymethyl cellulose, hydroxyethyl cellulose, hydroxypropyl cellulose, hydroxybutyl cellulose, hydroxypropylmethyl cellulose, carboxymethyl cellulose, hyaluronic acid, chondroitin-4-sulfate, chondroitin-6-sulfate, keratan sulfate, alginic acid, heparin, heparan sulfate, chitin, chitosan, salts thereof, derivatives thereof and mixtures thereof. Polyvinyl alcohol and/or starch are preferred in particular.

In a particularly preferred embodiment, the at least one layer, in particular the internal and/or external layers, more preferably the internal and external layers, of the wrapping comprises/comprise, in particular is/are made of, polyvinyl alcohol as a water-soluble component. In general, the polyvinyl alcohol may have a molecular weight from 5000 to 200000 g/mol, in particular from 10000 to 100000 g/mol. The polyvinyl alcohol of the external layer may preferably be a polyvinyl alcohol having a higher molecular weight than the polyvinyl alcohol of the internal layer. More preferable, the polyvinyl alcohol of the internal layer may have a molecular weight from 5000 to 150000 g/mol, in particular from 10000 to 100000 g/mol, whereas the polyvinyl alcohol of the external layer may preferably have a molecular weight from 40000 to 200000 g/mol, in particular from 70000 to 150000 g/mol. Further, the polyvinyl alcohol of the external layer may have a higher degree of a hydrolyzation than the polyvinyl alcohol of the internal layer. Furthermore, the polyvinyl alcohol of the external layer may have a higher degree of a cross-linking than the polyvinyl alcohol of the internal layer. Alternatively, the polyvinyl alcohol of the internal layer may be not cross-linked, whereas the polyvinyl alcohol of the external layer may be cross-linked, in particular physically cross-linked, preferably as a result of a sequence of freeze-thaw-cycles. Further, only the polyvinyl alcohol of the external layer may be dried, in particular thermally dried. More preferably, only the polyvinyl alcohol of the external layer may be crystallized, in particular thermally crystallized.

According to a further embodiment, the external layer comprises additives, preferably water proofness enhancing additives. More specifically, the additives may be binders and/or thickeners, and may be selected for example from the group consisting of cellulose, methyl cellulose, hydroxypropyl cellulose, hydroxypropylmethyl cellulose, ethylmethyl cellulose, carboxymethyl cellulose, salts thereof, derivatives thereof and mixtures thereof. The additives may be present in the external layer in a proportion from 0,1 to 50 % by weight, in particular from 1 to 30 % by weight, preferably from 3 to 10 % by weight, based on the total weight of the external layer.

In a further embodiment, the external layer may comprise, in particular may be made of, a bioresorbable, in particular hydrolyzable, preferably an in vivo hydrolyzable, component. The component may be a polyhydroxy alkanoate or a copolymer thereof. Preferably, the component may be selected from the group consisting of polylactide, polyglycolide, poly-ε-caprolactone, polytrimethylene carbonate, poly-para-dioxanone, poly-3-hydroxybutyrate, poly-4-hydroxybutyrate, copolymers thereof and mixtures or blends thereof. Within the scope of the present invention, the expression "copolymer" encompasses a polymer being composed of at least two different monomer units and, thus may also encompass terpolymers, tetrapolymers or the like. Furthermore, the expression "copolymer" as used according to the present invention may encompass statistical or random copolymers, alternating copolymers, block copolymers, graft polymers, atactic copolymers, isotactic copolymers and/or syndiotactic copolymers.

In another embodiment, the external layer of the wrapping may be water-repellent or hydrophobic. By that means, the water proofness of the external layer may be improved as compared to that of the internal layer. Preferably, the external layer comprises a water-repellent or hydrophobic component. The expression "a water-repellent or hydrophobic component" is preferably to comprehend not only a single water-repellent or hydrophobic component, but also multiple water-repellent or hydrophobic components, i.e. at least two water-repellent or hydrophobic components, according to the invention.

In another embodiment, the wrapping is water-repellent or hydrophobic in-between the internal and external layers. In particular, the wrapping comprises a water-repellent or hydrophobic component, preferably in the form of a water-repellent or hydrophobic layer or partition, interposed between the internal and external layers.

The water-repellent or hydrophobic component may preferably be selected from the group consisting of polyethylene oxide and/or polyethylene oxide copolymers, poloxamers and/or poloxamer copolymers, poloxamines and/or poloxamine copolymers, paraffin oils, fatty acid salts, in particular fatty acid salts of alkali metals and/or earth alkali metals, like calcium stearate, for example, and mixtures thereof.

In a further embodiment, the internal and external layers may be bonded together by means of welding, co-extruding, gluing or the like.

In a further embodiment, the wrapping may comprise additional layers, in particular between the internal and the external layers. As to the features and advantages of said additional layers, reference is made to the complete description, in particular in relation to the internal and external layers of the wrapping, as mentioned above. According to the invention, the layers of the wrapping may form a gradient as to their characteristics of water-solubility, degree of hydrolyzation, degree of cross-linking, degree of crystallinity, layer thickness and/or as to molecular weights of water-soluble components included in the layers, for example. It is preferred according to the invention, that water-solubility, degree of hydrolyzation, degree of cross-linking, degree of crystallinity, layer thickness and/or molecular weights exhibit a gradual increase from the internal layer to the external layer of the wrapping.

In principle, it may also be within the scope of the present invention that the at least one layer of the wrapping comprises areas having different water-solubility characteristics. In this regard it is preferable, that an internal side of the at least one water-soluble layer, preferably immediately facing the absorbent body, has a superior water-solubility than an external side of the at least one water-soluble layer. For instance, the external side may have a higher degree of cross-linking, a higher degree of hydrolyzation and/or a higher degree of crystallinity than the internal side. With respect to further features and advantages, in particular regarding the realization of different water-solubility characteristics, reference is made in its entirety to the previous description.

In another embodiment, the wrapping, in particular the at least one water-soluble layer, preferably the internal and/or external layers, preferably the internal and external layers, may be designed as a film or foil, preferably as a tubular film or foil. More specifically, the wrapping may be designed as a co-extruded film or foil, in particular a co-extruded tubular film or foil. Using said procedure, the production of an absorbent body packed up in a tubular film may be achieved in a particularly easy way. However, according to the invention, the internal and/or external layers of the wrapping may equally be provided in a means other than a film or foil, like in the form of a membrane or nonwoven layer, for example. Preferred is that the external, in particular only the external layer, may be present as a sprayed layer.

A further aspect of the present invention is related to a medical product, in particular for removing body liquids such as wound secretions from human and/or animal body cavities, comprising an absorbent body, a drainage tube and a wrapping surrounding the absorbent body and keeping the absorbent body in a compressed form, wherein the wrapping comprises at least two, in particular two, water-soluble components differing in their water-solubility.

The wrapping may be in particular made of the at least two water-soluble components.

More specifically, the water-soluble components may differ with respect to their molecular weight, degree of hydrolyzation, degree of cross-linking and/or degree of crystallinity. In particular, one water-soluble component may be cross-linked, particularly chemically and/or physically cross-linked, whereas the other water-soluble component is preferably uncross-linked.

Preferably, the more water-soluble component, in particular the water-soluble component having the lower molecular weight, the lower degree of hydrolyzation, the lower degree of cross-linking and/or the lower degree of crystallinity, is comprised as a predetermined breaking structure or predetermined breaking site, for example in the form of perforations, lines, spots or the like, in a layer made of the less water-soluble component, in particular of the water-soluble component having the higher molecular weight, the higher degree of hydrolyzation, the higher degree of cross-linking and/or the higher degree of crystallinity. Further, it is preferred according to the invention that the more water-soluble component is provided on the internal side of the wrapping.

In general, the water-soluble components may be made from the same or a different material. However, it may be preferred that the water-soluble components are made from the same material. More preferably, the material differs in view of its molecular weight, degree of hydrolyzation, degree of cross-linking and/or degree of crystallinity.

In a preferred embodiment, the water-soluble components may be made of a material selected from the group consisting of polyvinyl alcohol, polysaccharides, like alkyl celluloses, hydroxyalkyl celluloses, carboxyalkyl celluloses and/or mucopolysaccharides, salts thereof, derivatives thereof and mixtures thereof. Particularly preferred are water-soluble components selected from the group consisting of polyvinyl alcohol, polyethylene glycol, gelatine, starch, amylose, amylopectin, dextran, methyl cellulose, hydroxymethyl cellulose, hydroxyethyl cellulose, hydroxypropyl cellulose, hydroxybutyl cellulose, hydroxypropylmethyl cellulose, carboxymethyl cellulose, hyaluronic acid, chondroitin-4-sulfate, chondroitin-6-sulfate, keratan sulfate, alginic acid, heparin, heparan sulfate, chitin, chitosan, salts thereof, derivatives thereof and mixtures thereof. Polyvinyl alcohol and/or starch are preferred in particular.

With respect to further details and advantages of the water-soluble components, reference is made in its entirety to the previous description.

The absorbent body is preferably designed deployable or expandable, in particular reversibly deployable or reversibly expandable.

In a preferred embodiment, the absorbent body in the compressed form has a volume reduced by from 10 to 90%, in particular from 20 to 80%, preferably from 40 to 70%, than without the wrapping. The absorbent body is preferably provided compressed in two dimensions. More specifically, the absorbent body may be provided in a radially compressed form.

The compressed absorbent body may in principle be provided in various shapes. For instance, starting from a basic shape, the absorbent body may be adapted in shape and size, in particular cut, for a body cavity to be treated. For example, the absorbent body may have a circular, oval, triangular, square, trapezoidal, rhomboid, pentagonal or five-sided, hexagonal, star- or cross-shaped cross section.

Furthermore, the absorbent body may be designed as a tube, hose, cylinder or the like.

The absorbent body is preferably designed to be porous, particularly preferably open-porous or open-pored. The absorbent body may in principle have a pore size of between 100 and 1500 µm, in particular 200 and 1000 µm, preferably 400 and 800 µm, more preferably 400 µm to 600 µm.

In another embodiment, the absorbent body and/or the wrapping comprise active agents, in particular selected from the group consisting of antimicrobial, antiseptic, disinfectant, growth-promoting, odour-inhibiting, anti-inflammatory active agents and mixtures thereof. For example, triclosan, polyhexamethylene biguanide (PHMB), copper, zinc, silver, gold, compounds thereof and/or salts thereof may be mentioned as antimicrobial active agents. The active agents may generally be provided in particulate form, particularly in the form of nano- and/or microparticles.

Generally, the absorbent body may be designed as a pad, a membrane, sponge or foam body. In a particularly preferred embodiment, the absorbent body is a sponge or foam body, preferably a sponge body. A sponge body particularly advantageously has a larger absorbent surface area.

The absorbent body in a further embodiment is made from a polymer, particularly a biocompatible polymer. The polymer may be a homo- or copolymer. The absorbent body is preferably made of a polymer selected from the group consisting of polypropylene, polyethylene, polyester such as polyethylene terephthalate, polypropylene terephthalate, polybutylene terephthalate, polyurethane, polyamide such as nylon 6, nylon 6.6, polyhydroxy alkanoate such as polylactide, polyglycolide, poly-ε-caprolactone, poly-3-hydroxybutyrate and/or poly-4-hydroxybutyrate, polytrimethylene carbonate, poly-para-dioxanone, copolymers thereof, derivatives thereof and mixtures or blends thereof. The absorbent body is particularly preferably made of a polyurethane or a polyurethane derivative, in particular polyurethane ether or copolymers thereof. The polyurethane may be an aliphatic polyurethane. The polyurethane is preferably a linear, aliphatic polyurethane. The polyurethane itself may be formed from macromolecular and/or low molecular weight aliphatic diols and aliphatic diisocyanates. Primarily polycarbonates, in particular 1,6-hexanediol polycarbonate, may be envisaged as macromolecular diols. For example, 2,2,4-trimethylhexanediol, 2,4,4-trimethylhexanediol and/or 1,4-butanediol may be used as low molecular weight diols. Preferably cycloaliphatic diisocyanates, in particular 4,4-dicyclohexylmethane diisocyanate or 1,4-cyclohexyl diisocyanate, may be envisaged as aliphatic diisocyanates. The polyurethane may furthermore be produced from different diols and/or diisocyanates. Polyurethane is particularly preferred as a material for the absorbent body owing to its biocompatibility.

In a further embodiment, the drainage tube is connected, in particular integrally, to the absorbent body. In the case of the drainage tube, distinction can generally be made between a proximal tube end (near the body of a patient) and a distal tube end (away from the body of a patient). The drainage tube is preferably connected to the absorbent body with its proximal end, whereas the distal end of the drainage tube is free and may for example be connected to a suction or vacuum source, in particular a suction or vacuum pump. With the aid of the suction or vacuum source, a negative pressure or suction of between 400 and 900 mbar (which approximately corresponds to from 300 mmHg to 675 mmHg), in particular 500 and 800 mbar (which approximately corresponds to from 375 mmHg to 600 mmHg) may be generated. Particularly rapid cleaning of infectious body cavities, and rapid granulation in the body cavities, are therefore possible.

The vacuum source may for example be a portable vacuum pump. In this way, the patient's mobility can be maintained during the treatment. In principle, the absorbent body may be provided for placement in a body cavity for several hours to several days. The absorbent body is typically changed every 8 to 72 hours. The quantities of liquid removed or discharged are normally gathered in collection containers intended for this, for example canisters or vacuum bottles. The collection containers are generally connected upstream of a suction or vacuum source and are in contact with it through suitable connection tubes. In order to avoid contaminating the suction or vacuum source, a sterile filter may be provided between the collection containers and the suction or vacuum source.

Preferably, the drainage tube comprises a portion, generally an end portion, invading or penetrating the absorbent body. In other words, it may be preferable if a portion, generally an end portion, of the drainage tube is enveloped, sheathed or jacketed by the absorbent body. For taking the drainage tube, in particular an end portion thereof, the absorbent body preferably has a recess, in particular a channel-like, tube-like or cylinder-like recess, having a diameter that usefully corresponds to the outer diameter of the drainage tube. The recess may be formed by cross- or star-shaped stamping (without material being removed).

More specifically, the portion that is enveloped by the absorbent body preferably comprises openings such as holes or perforations in the tube wall. Thus, a better liquid communication between the absorbent body and the drainage tube is facilitated.

Furthermore, the absorbent body may in particular be formed integrally on the drainage tube. For example, the absorbent body may be adhesively bonded, stitched or welded to the drainage tube and/or expansion-moulded onto the drainage tube.

The drainage tube may generally be formed from a plastic or synthetic material such as polyethylene, polypropylene, polyvinyl chloride and/or polyurethane.

In a further embodiment, the medical product, in particular the absorbent body, may additionally comprise a flushing or rinsing tube. With the aid of the flushing tube, the absorbent body can be flushed. The flushing or rinsing liquid used may for example be a sodium chloride solution, buffer solution, anti-inflammatory, odour-inhibiting and/or antimicrobial solution. As an alternative, and more preferably, the absorbent body may also be flushed and delivered with the aforementioned liquids via the drainage tube.

The absorbent body according to the invention may furthermore be provided in a sterilized form and, in particular, in a packaged form. In this regard, in principle all sterilizing and packaging methods familiar to the person skilled in the art may be envisaged.

Further features and advantages of the invention may be found in the following description of preferred embodiments in the form of figure descriptions including the corresponding figures and an example. Individual features may respectively be implemented on their own, or several together in combination with one another. The figure descriptions including the corresponding figures and the example merely serve to explain the present invention without in any way restricting it thereto.

### Description of the Figure

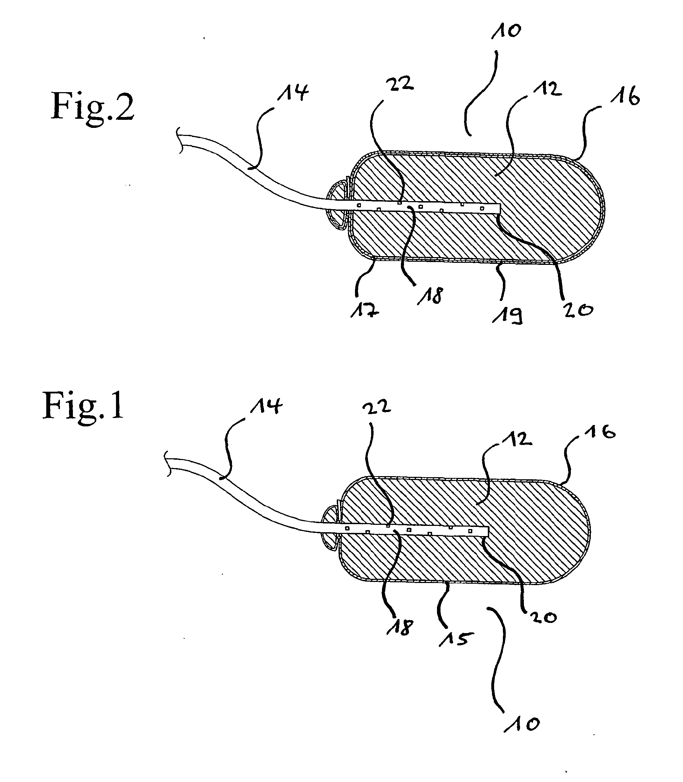

Figure 1 is a schematic illustration of a medical product 10 according to the present invention. The medical product 10 comprises an absorbent body 12, a drainage tube 14 and a wrapping 16. The wrapping 16 completely surrounds the absorbent body 12, except from the area where the drainage tube 14 emerges out of or protrudes from the absorbent body 12. The wrapping 16 comprises a water soluble layer 15 and keeps the absorbent body 12, which is preferably designed as a reversibly expandable body, in a compressed form. By way of example, the water-soluble layer 15 may comprise polyvinyl alcohol (PVA).

The sponge body 12, as depicted in figure 1, may have an essentially cylindrical or tubular configuration. Further, the absorbent body 12 is preferably designed as a sponge body. Advantageously, the absorbent body 12 has an open-porous structure. Usually the absorbent body 12 is made of a biocompatible polymer such as polyurethane.

Expediently, the drainage tube 14 comprises a portion 18 that invades the absorbent body 12. For invasion of portion 18, the absorbent body 12 advantageously has a corresponding recess 20. In order to allow for a better liquid transfer from the absorbent body 12 into the drainage tube 14, portion 18 may have openings 22 such as holes or perforations breaking through the tube wall of portion 18.

After correct placement of the medical product 10 into a body cavity to be treated, the absorbent body 12 may be flushed from the inside via the drainage tube 14, thereby starting the dissolution of the water-soluble layer 15. With proceeding dissolution of the layer 15, the wrapping 16 is increasingly decomposed and weakened. Due to that, the rest of the wrapping 16 may finally yield, in particular may rip open, thereby facilitating the unfolding or expansion of the absorbent body 12.

Figure 2 is a further schematic illustration of a medical product 10 according to the present invention. The medical product 10 comprises an absorbent body 12, and a drainage tube 14 as depicted in figure 1 and as described in the related description above. In this regard, reference is made in its entirety to the description of figure 1. Further, the medical product 10 comprises also a wrapping 16 which completely surrounds the absorbent body 12, except from the area where the drainage tube 14 emerges out of or protrudes from the absorbent body 12. The wrapping 16 also keeps the absorbent body 12, which is preferably designed as a reversibly expandable body, in a compressed form. However, in this embodiment, the wrapping 16 comprises an internal layer 17 and an external layer 19. Preferably, the internal layer 17 has a water-solubility superior to that of the external layer 19. By way of example, the internal layer 17 and the external layer 19 may each comprise polyvinyl alcohol (PVA) as a water-soluble component. More specifically, the polyvinyl alcohol of the internal layer 17 may have a lower molecular weight, a lower degree of hydrolyzation, a lower degree of cross-linking and/or a lower degree of crystallinity than the polyvinyl alcohol of the external layer 19. Further, unlike the polyvinyl alcohol of the external layer 19, the polyvinyl alcohol of the internal layer 17 may be uncross-linked and/or not dried, in particular not thermally dried.

After correct placement of the medical product 10 into a body cavity to be treated, the absorbent body 12 may be flushed from the inside via the drainage tube 14, thereby starting the dissolution of the internal layer 17. With proceeding dissolution of the internal layer 17, the wrapping 16 is increasingly decomposed and weakened. Due to that, the rest of the wrapping 16, in particular the external layer 19, may finally yield, in particular may rip open, thereby facilitating the unfolding or expansion of the absorbent body 12.

Having regard to a wrapping whose internal layer has a water-solubility superior to that of an external layer thereof, several advantages may be achieved:

Due to the lower water-solubility of the external layer, an undesired premature decomposition and weakening of the wrapping from the outside thereof caused by body liquids present in a body cavity, and thus an undesired premature unfolding or expansion of the absorbent body may be avoided.

Due to the different water-solubility characteristics of the internal and external layers, a time-controlled or time-directed decomposition and weakening of the wrapping, and thus a time-controlled or time-directed unfolding or expansion of the absorbent body may be realized. Thus, the operator may exercise influence on the decomposition, and thus on the unfolding of the absorbent body by flushing the absorbent body from the inside via the drainage tube, thereby initiating the dissolution of the internal layer.

Due to the different water-solubility characteristics of the internal and external layers, the operator may have enough time to replace the absorbent body within a body cavity so as to achieve a better therapeutic effect.

Due to the water-soluble characteristics of the internal and external layers, a cumbersome removal of the wrapping from the absorbent body is no longer required by the operator.

### Example

Starting from a solution including a proportion of polyvinyl alcohol (PVA) of 10 % by weight and a proportion of carboxymethyl cellulose of 1.5 % by weight, based on the total weight of the solution, films having a thickness of about 50 µm were prepared using a coating knife. The films were dried and gamma-ray sterilized. A portion of the films were repeatedly coated with a chloroform solution of polylactide (PLLA).

Subsequently, the films were welded to a tubular film using a bar welding apparatus. In case of coated films, the welding was performed in a manner that the coating was present on the exterior side of the tubular film.

Subsequently, polyurethane sponge cylinders having a diameter of 24 mm and a drainage tube were drawn into the tube films under compression of the cylinders, while the drainage tube protruded from the wrapping.

Part of the packed sponge cylinders were subject to a flow of water on the outside, while the time up to dissolution or up to yielding and ripping of the film and expansion of the sponge cylinders was recorded. Another part of the packed sponge cylinders was subject to a flow of water from the inside. As well, the time up to expansion of the sponge cylinders was recorded. The thereby obtained results are listed in the table 1 below:

**Table 1**

| Water outside [sec] uncoated | Water inside [sec] uncoated | Water outside PLLA coatings (external) [sec] | Water inside PLLA coatings (external) [sec] |
|---|---|---|---|
| 40 | 55 | 125 | 59 |
| 35 | 49 | 133 | 77 |
| 38 | 68 | 139 | 69 |

## Claims

1. A medical product (10), in particular for removing body liquids such as wound secretions from human and/or animal body cavities, comprising an absorbent body (12), a drainage tube (14), and a wrapping (16) surrounding the absorbent body (12), **characterized in that** the wrapping (16) keeps the absorbent body (12) in a compressed form and comprises at least one water-soluble layer (15).

2. A medical product (10) according to claim 1, **characterized in that** the wrapping (16) comprises an internal layer (17), preferably immediately surrounding the absorbent body (12), and an external layer (19), wherein at least one of the layers is water-soluble.

3. The medical product (10) according to claim 2, **characterized in that** the internal layer (17), in particular only the internal layer (17), is water-soluble, preferably comprises a water-soluble component.

4. The medical product (10) according to claim 2 or 3, **characterized in that** the internal and/or external layers (17; 19) are water-soluble, preferably each comprise a water-soluble component.

5. The medical product (10) according to any of claims 2 to 4, **characterized in that** the internal and external layers (17; 19) each have a different water-solubility, preferably each comprises a water-soluble component differing in water-solubility, wherein the internal layer (17) preferably has a water-solubility superior to that of the external layer (19), preferably the internal layer (17) comprises a water-soluble component having a water-solubility superior to that of a water-soluble component of the external layer (19).

6. The medical product (10) according to any of claims 2 to 5, **characterized in that** the external layer (19) comprises a water-soluble component having a higher molecular weight than a water-soluble component of the internal layer (17).

7. The medical product (10) according to any of claims 2 to 6, **characterized in that** the external layer (19) has a higher degree of hydrolyzation than the internal layer (17), preferably the external layer (19) comprises a water-soluble component having a higher degree of hydrolyzation than a water-soluble component of the internal layer (17).

8. The medical product (10) according to any of claims 2 to 7, **characterized in that** the external layer (19), in particular only the external layer (19), preferably a water-soluble component of the external layer (19), is crosslinked.

9. The medical product (10) according to any of claims 2 to 8, **characterized in that** the external layer (19), in particular only the external layer (19), is crystallized, in particular thermally crystallized.

10. The medical product (10) according to any of claims 2 to 9, **characterized in that** the internal layer (17) has a larger layer thickness than the external layer (19).

11. The medical product (10) according to any of the preceding claims, **characterized in that** the at least one water-soluble layer (15), in particular the internal and/or external layers (17; 19), comprises a water-soluble component made of a material selected from the group consisting of polyvinyl alcohol, polyethylene glycol, gelatine, starch, amylose, amylopectin, dextran, methyl cellulose, hydroxymethyl cellulose, hydroxyethyl cellulose, hydroxypropyl cellulose, hydroxybutyl cellulose, hydroxyethylmethyl cellulose, hydroxypropylmethyl cellulose, carboxymethyl cellulose, hyaluronic acid, chondroitin-4-sulfate, chondroitin-6-sulfate, keratin sulfate, alginic acid, heparin, heparan sulfate, chitin, chitosan, salts thereof, derivatives thereof and mixtures thereof.

12. The medical product (10) according to any of claims 2 to 11, **characterized in that** the external layer (19) comprises a bioresorbable, in particular in vivo hydrolyzable component, preferably selected from the group consisting of polylactide, polyglycolide, poly-ε-caprolactone, polytrimethylene carbonate, poly-para-dioxanone, poly-3-hydroxybutyrate, poly-4-hydroxybutyrate, copolymers thereof and mixtures or blends thereof.

13. The medical product (10) according to any of claims 2 to 12, **characterized in that** the external layer (19), in particular only the external layer (19), comprises a water proofness enhancing additive, in particular a binder and/or thickener, preferably selected from the group consisting of cellulose, methyl cellulose, hydroxypropyl cellulose, hydroxypropylmethyl cellulose, carboxymethyl cellulose, salts thereof, derivatives thereof and mixtures thereof.

14. The medical product (10) according to any of claims 2 to 13, **characterized in that** the external layer (19) is water-repellent or hydrophobic, preferably comprises a water-repellent or hydrophobic component.

15. The medical product (10) according to any of claims 2 to 14, **characterized in that** the wrapping (16) comprises a water-repellent or hydrophobic component, preferably in the form of a water-repellent or hydrophobic layer, interposed between the internal and external layers (17; 19).

16. The medical product (10) according to claim 14 or 15, **characterized in that** the water-repellent or hydrophobic component is selected from the group consisting of polyethylene oxide or polyethylene oxide copolymers, poloxamers or poloxamer copolymers, poloxamines or poloxamine copolymers, paraffin oils, fatty acid salts, in particular fatty acid salts of alkali metals and/or earth alkali metals, like calcium stearate for example, and mixtures thereof.

17. The medical product (10) according to any of the preceding claims, **characterized in that** the wrapping (16), in particular the at least one water-soluble layer (15), more preferably the internal and/or external layers (17; 19), is present as a film or foil, preferably as a tubular film or tubular foil.

## Patentansprüche

1. Medizinisches Produkt (10), insbesondere zum Entfernen von Körperflüssigkeiten wie Wundsekreten aus menschlichen und/oder tierischen Körperhöhlen, umfassend einen Saugkörper (12), einen Drainageschlauch (14) und eine Umhüllung (16), die den Saugkörper (12) umgibt, **dadurch gekennzeichnet, dass** die Umhüllung (16) den Saugkörper (12) in einer komprimierten Form hält und mindestens eine wasserlösliche Schicht (15) umfasst.

2. Medizinisches Produkt (10) nach Anspruch 1, **dadurch gekennzeichnet, dass** die Umhüllung (16) eine innere Schicht (17), die bevorzugt den Saugkörper (12) unmittelbar umgibt, und eine äußere Schicht (19) umfasst, wobei mindestens eine der beiden Schichten wasserlöslich ausgebildet ist.

3. Medizinisches Produkt (10) nach Anspruch 2, **dadurch gekennzeichnet, dass** die innere Schicht (17), insbesondere nur die innere Schicht (17), wasserlöslich ausgebildet ist, bevorzugt eine wasserlösliche Komponente umfasst.

4. Medizinisches Produkt (10) nach Anspruch 2 oder 3, **dadurch gekennzeichnet, dass** die innere und/oder die äußere Schicht (17; 19) wasserlöslich ausgebildet sind, vorzugsweise jeweils eine wasserlösliche Komponente umfassen.

5. Medizinisches Produkt (10) nach einem der Ansprüche 2 bis 4, **dadurch gekennzeichnet, dass** die innere und die äußere Schicht (17; 19) jeweils eine unterschiedliche Wasserlöslichkeit aufweisen, bevorzugt jeweils eine wasserlösliche Komponente aufweisen, die sich in ihrer Wasserlöslichkeit unterscheiden, wobei die innere Schicht (17) bevorzugt eine höhere Wasserlöslichkeit besitzt als die äußere Schicht (19), die innere Schicht (17) bevorzugt eine wasserlösliche Komponente umfasst, die eine höhere Wasserlöslichkeit besitzt als die wasserlösliche Komponente der äußeren Schicht (19).

6. Medizinisches Produkt (10) nach einem der Ansprüche 2 bis 5, **dadurch gekennzeichnet, dass** die äußere Schicht (19) eine wasserlösliche Komponente umfasst, die ein höheres Molekulargewicht besitzt als eine wasserlösliche Komponente der inneren Schicht (17).

7. Medizinisches Produkt (10) nach einem der Ansprüche 2 bis 6, **dadurch gekennzeichnet, dass** die äußere Schicht (19) einen höheren Hydrolysegrad aufweist als die innere Schicht (17), bevorzugt die äußere Schicht (19) eine wasserlösliche Komponente umfasst, die einen höheren Hydrolysegrad aufweist als eine wasserlösliche Komponente der inneren Schicht (17).

8. Medizinisches Produkt (10) nach einem der Ansprüche 2 bis 7, **dadurch gekennzeichnet, dass** die äußere Schicht (19), insbesondere nur die äußere Schicht (19), bevorzugt eine wasserlösliche Komponente der äußeren Schicht (19), vernetzt vorliegt.

9. Medizinisches Produkt (10) nach einem der Ansprüche 2 bis 8, **dadurch gekennzeichnet, dass** die äußere Schicht (19), insbesondere nur die äußere Schicht (19), kristallisiert vorliegt, insbesondere thermisch kristallisiert vorliegt.

10. Medizinisches Produkt (10) nach einem der Ansprüche 2 bis 9, **dadurch gekennzeichnet, dass** die innere Schicht (17) eine höhere Schichtdicke aufweist als die äußere Schicht (19).

11. Medizinisches Produkt (10) nach einem der vorhergehenden Ansprüche **dadurch gekennzeichnet, dass** die mindestens eine wasserlösliche Schicht (15), insbesondere die innere und/oder die äußere Schicht (17; 19), eine wasserlösliche Komponente umfasst, die aus einem Material gebildet ist, das ausgewählt ist aus der Gruppe bestehend aus Polyvinylalkohol, Polyethylenglycol, Gelatine, Stärke, Amylose, Amylopektin, Dextran, Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Hyaluronsäure, Chondroitin-4-Sulfat, Chondroitin-6-Sulfat, Keratinsulfat, Alginsäure, Heparin, Heparansulfat, Chitin, Chitosan, Salzen davon, Derivaten davon und Mischungen davon.

12. Medizinisches Produkt (10) nach einem der Ansprüche 2 bis 11, **dadurch gekennzeichnet, dass** die äußere Schicht (19) eine bioresorbierbare, insbesondere *in vivo* hydrolysierbare Komponente umfasst, die bevorzugt ausgewählt ist aus der Gruppe bestehend aus Polylactid, Polyglycolid, Poly-ε-Caprolacton, Polytrimethylencarbonat, Poly-para-Dioxanon, Poly-3-Hydroxybutyrat, Poly-4-Hydroxybutyrat, Copolymeren davon und Mischungen oder Blends davon.

13. Medizinisches Produkt (10) nach einem der Ansprüche 2 bis 12, **dadurch gekennzeichnet, dass** die äußere Schicht (19), insbesondere nur die äußere Schicht (19), einen deren Wasserbeständigkeit erhöhenden Zusatzstoff, insbesondere ein Bindemittel und/oder ein Verdickungsmittel, vorzugsweise ausgewählt aus der Gruppe bestehend aus Cellulose, Methylcellulose, Hydroxypropylcellulose, Hydroxypropylmethylcellulose, Carboxymethylcellulose, Salze davon, Derivate davon und Mischungen davon, umfasst.

14. Medizinisches Produkt (10) nach einem der Ansprüche 2 bis 13, **dadurch gekennzeichnet, dass** die äußere Schicht (19) wasserabweisend oder hydrophob ausgebildet ist, vorzugsweise eine wasserabweisende oder hydrophobe Komponente umfasst.

15. Medizinisches Produkt (10) nach einem der Ansprüche 2 bis 14, **dadurch gekennzeichnet, dass** die Umhüllung (16) eine wasserabweisende oder hydrophobe Komponente umfasst, vorzugsweise in Form einer wasserabweisenden oder hydrophoben Schicht, die zwischen die innere und äußere Schicht (17; 19) eingesetzt ist.

16. Medizinisches Produkt (10) nach Anspruch 14 oder 15, **dadurch gekennzeichnet, dass** die wasserabweisende oder hydrophobe Komponente ausgewählt ist aus der Gruppe bestehend aus Polyethylenoxid oder Polyethylenoxidcopolymeren, Poloxameren oder Poloxamer-Copolymeren, Poloxaminen oder Poloxamin-Copolymeren, Paraffinölen, Fettsäuresalzen, insbesondere Alkalimetall- und/oder Erdalkalimetallfettsäuresalzen, wie beispielsweise Calciumstearat, und Mischungen davon.

17. Medizinisches Produkt (10) nach einem der vorhergehenden Ansprüche, **dadurch gekennzeichnet, dass** die Umhüllung (16), insbesondere die mindestens eine wasserlösliche Schicht (15), besonders bevorzugt die innere und/oder die äußere Schicht (17; 19), in Form eines Films oder einer Folie, vorzugsweise als schlauchförmiger Film oder Schlauchfolie vorliegt.

## Revendications

1. Produit médical (10), en particulier pour enlever des liquides corporels tels que des exsudats de plaies de cavités corporelles humaines et/ou animales, comprenant un corps absorbant (12), un drain tubulaire (14), et une enveloppe (16) entourant le corps absorbant (12), **caractérisé en ce que** l'enveloppe (16) maintient le corps absorbant (12) dans une forme comprimée et comprend au moins une couche hydrosoluble (15).

2. Produit médical (10) selon la revendication 1, **caractérisé en ce que** l'enveloppe (16) comprend une couche interne (17), située de préférence immédiatement autour du corps absorbant (12), et une couche externe (19), au moins une des couches étant hydrosoluble.

3. Produit médical (10) selon la revendication 2, **caractérisé en ce que** la couche interne (17), en particulier seulement la couche interne (17), est hydrosoluble, et comprend préférablement un constituant hydrosoluble.

4. Produit médical (10) selon la revendication 2 ou 3, **caractérisé en ce que** les couches interne et/ou externe (17 ; 19) sont hydrosolubles, chacune comprenant préférablement un constituant hydrosoluble.

5. Produit médical (10) selon l'une quelconque des revendications 2 à 4, **caractérisé en ce que** les couches interne et externe (17 ; 19) ont chacune une différente hydrosolubilité, chacune comprenant préférablement un constituant hydrosoluble ayant une différente hydrosolubilité, dans lequel la couche interne (17) a préférablement une hydrosolubilité supérieure à celle de la couche externe (19), la couche interne (17) comprenant préférablement un constituant hydrosoluble ayant une hydrosolubilité supérieure à celle d'un constituant hydrosoluble de la couche externe (19).

6. Produit médical (10) selon l'une quelconque des revendications 2 à 5, **caractérisé en ce que** la couche externe (19) comprend un constituant hydrosoluble ayant une masse moléculaire supérieure à celle d'un constituant hydrosoluble de la couche interne (17).

7. Produit médical (10) selon l'une quelconque des revendications 2 à 6, **caractérisé en ce que** la couche externe (19) a un degré d'hydrolysation supérieur à celui de la couche interne (17), la couche externe (19) comprenant préférablement un constituant hydrosoluble ayant un degré d'hydrolysation supérieur à celui d'un constituant hydrosoluble de la couche interne (17).

8. Produit médical (10) selon l'une quelconque des revendications 2 à 7, **caractérisé en ce que** la couche externe (19), en particulier seulement la couche externe (19), préférablement un constituant hydrosoluble de la couche externe (19), est réticulé(e).

9. Produit médical (10) selon l'une quelconque des revendications 2 à 8, **caractérisé en ce que** la couche externe (19), en particulier seulement la couche externe (19), est cristallisée, en particulier cristallisée thermiquement.

10. Produit médical (10) selon l'une quelconque des revendications 2 à 9, **caractérisé en ce que** la couche interne (17) a une épaisseur de couche supérieure à celle de la couche externe (19).

11. Produit médical (10) selon l'une quelconque des revendications précédentes, **caractérisé en ce que** ladite au moins une couche hydrosoluble (15), en particulier la couche interne et/ou la couche externe (17 ; 19), comprend un constituant hydrosoluble qui se compose d'un matériau sélectionné dans le groupe constitué de l'alcool polyvinylique, du polyéthylène glycol, de la gélatine, de l'amidon, de l'amylose, de l'amylopectine, du dextrane, de la méthylcellulose, de l'hydroxyméthylcellulose, de l'hydroxyéthylcellulose, de l'hydroxypropylcellulose, de l'hydroxybutylcellulose, de l'hydroxy-éthylméthylcellulose, de l'hydroxypropylméthyl-cellulose, de la carboxyméthylcellulose, de l'acide hyaluronique, du 4-sulfate de chondroïtine, du 6-sulfate de chondroïtine, du sulfate de kératine, de l'acide alginique, de l'héparine, du sulfate d'héparane, de la chitine, du chitosane, de sels de ceux-ci, de dérivés de ceux-ci et de mélanges de ceux-ci.

12. Produit médical (10) selon l'une quelconque des revendications 2 à 11, **caractérisé en ce que** la couche externe (19) comprend un constituant biorésorbable, en particulier un constituant hydrolysable in vivo, préférablement sélectionné dans le groupe constitué du polylactide, du polyglycolide, de la poly-ε-caprolactone, du carbonate de polytriméthylène, de la poly-para-dioxanone, du poly-3-hydroxybutyrate, du poly-4-hydroxybutyrate, de copolymères de ceux-ci et de mélanges ou de mélanges homogènes de ceux-ci.

13. Produit médical (10) selon l'une quelconque des revendications 2 à 12, **caractérisé en ce que** la couche externe (19), en particulier seulement la couche externe (19), comprend un additif augmentant la résistance à l'eau, en particulier un liant et/ou un épaississant, préférablement sélectionné dans le groupe constitué de la cellulose, de la méthylcellulose, de l'hydroxypropylcellulose, de l'hydroxypropylméthylcellulose, de la carboxyméthylcellulose, de sels de celles-ci, de dérivés de celles-ci et de mélanges de celles-ci.

14. Produit médical (10) selon l'une quelconque des revendications 2 à 13, **caractérisé en ce que** la couche externe (19) est hydrofuge ou hydrophobe, et comprend préférablement un constituant hydrofuge ou hydrophobe.

15. Produit médical (10) selon l'une quelconque des revendications 2 à 14, **caractérisé en ce que** l'enveloppe (16) comprend un constituant hydrofuge ou hydrophobe, préférablement sous la forme d'une couche hydrofuge ou hydrophobe, interposée entre les couches interne et externe (17 ; 19).

16. Produit médical (10) selon la revendication 14 ou 15, **caractérisé en ce que** le constituant hydrofuge ou hydrophobe est sélectionné dans le groupe constitué de l'oxyde de polyéthylène ou de copolymères d'oxyde de polyéthylène, de poloxamères ou de copolymères de poloxamères, de poloxamines ou de copolymères de poloxamines, d'huiles de paraffine, de sels d'acides gras, en particulier des sels d'acides gras de métaux alcalins et/ou de métaux alcalino-terreux, tels que par exemple le stéarate de calcium, et de mélanges de ceux-ci.

17. Produit médical (10) selon l'une quelconque des revendications précédentes, **caractérisé en ce que** l'enveloppe (16), en particulier ladite au moins une couche hydrosoluble (15), plus préférablement la couche interne et/ou la couche externe (17 ; 19), est présente sous la forme d'un film ou d'une feuille, préférablement sous la forme d'un film tubulaire ou d'une feuille tubulaire.
